# EUROPEAN PATENT SPECIFICATION

(11) **EP 1 194 102 B1**
(45) Date of publication and mention of the grant of the patent: **23.11.2005**
(21) Application number: 00946601.2
(22) Date of filing: 19.06.2000
(51) Int. Cl.: A61F 13/56

(54) **ABSORBENT ARTICLE PROVIDED WITH A BELT**
ABSORBIERENDER ARTIKEL MIT EINEM GÜRTEL
ARTICLE ABSORBANT AVEC CEINTURE

(30) Priority: 29.06.1999 SE 9902448
(43) Date of publication of application: 10.04.2002
(73) Proprietor: SCA Hygiene Products AB, 405 03 Göteborg (SE)
(72) Inventor: FORGAR, Monica, S-417 28 Göteborg (SE)
(74) Representative: Egeröd, Lisbeth
(86) International application number: PCT/SE2000/001287
(87) International publication number: WO 2001/000129

(56) References cited:
- EP-A2- 0 409 307
- EP-A2- 0 893 115
- WO-A1-91/04724
- WO-A1-97/34037
- US-A- 5 706 524

## Description

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a belt attached to or intended to be attached to the rear portion of the article and which are intended to be fastened together around the waist of the wearer by belt attachment means and where said front portion is provided with front portion attachment means intended to be attached to the belt portions and to the front portion of the article, in such a way that the article will assume a pantlike shape, where the belt forms a part of the waist portion of the pant.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook-and-loop fasteners which directly attach the front and rear portions of the absorbent article to each other. It is further known, through e g EP-A-0 287 388, EP-A-0 409 307, EP-A-0 605 012 and FR-A-2 586 558, to attach the front and rear portions of the article by means of a belt, at which the possibilities to adjust the fit are improved. Depending on type of attachment means different types of attachment surfaces are used which are intended to cooperate with the attachment means.

US-A-5,706,524 discloses a disposable undergarment waistband comprising first and second layers of nonwoven material and elastic strips held therebetween. Fasteners are secured to each end of the waistband. A pair of waistbands are by the fasteners detachably connectable to mating fasteners on the upper peripheral portions of the undergarment panel.

WO 91/04724 discloses a diaper having an elastic band secured in a stretched state between two material layers. The elastic band may be attached along the rear waist line of the diaper.

WO 97/34037 discloses a nonwoven laminate having at least one layer of meltblown elastic fibers bonded on either side with a layer of soft non-elastic fibers. The laminate is useful in personal care products, infection control products, garments and protective covers.

EP-A-893 115 discloses a diaper having male fasteners at rear flaps, and wherein a part of the outer surface of the front flap is a nonwoven material serving as a female fastener.

None of these documents do however refer to an absorbent article having a belt intended to be fastened together around the waist of the wearer.

A problem with belts of said type is that they easily cause skin irritations to the user, due to that the belt is in direct contact with the skin of the wearer and has to be tightened relatively strongly in order to have a satisfactory fit and security against leakage of the diaper or incontinence guard. By the tight contact and friction between the belt and the skin there will be a mechanic wear of the skin which gives rise to irritation and even skin injuries.

### The object and most important features of the invention

The object of the invention is to provide a belt for absorbent articles which is kind to the skin and by that does not give rise to skin irritations and injuries and in which the belt material per se can cooperate with different kinds of attachment means for providing a flexible fit of the absorbent article. This has been solved by the fact that the belt comprises a flexible laminate comprising an elastic carrier material, which at least on a part of the side intended to form the outside of the belt is covered with a nonwoven material having a basis weight of at least 15 and preferably at least 20 g/m² and which may serve as attachment surface for attachment means both in the form of adhesive tape and hook-and-loop type fasteners, at which the attachment surface also admits refastening of said attachment means.

The nonwoven material which is intended to form the outside of the belt mainly comprises continuous filaments, such as spunbond material or meltblown material.

The elastic carrier material can be an elastic film, which preferably is perforated, or an elastic nonwoven material.

The elastic carrier material is preferably on the side intended to form the inside of the belt covered with a soft skin friendly nonwoven material.

According to a preferred embodiment at least the nonwoven material that is intended to form the outside of the belt is joined with the elastic material in a pattern so that a three-dimensional surface structure is obtained at least on one side of the laminate. By that an improved attachment surface is obtained for a hook-and-loop type fastener.

### Description of drawings

The invention will in the following be closer described with reference to an embodiment shown in the accompanying drawings.

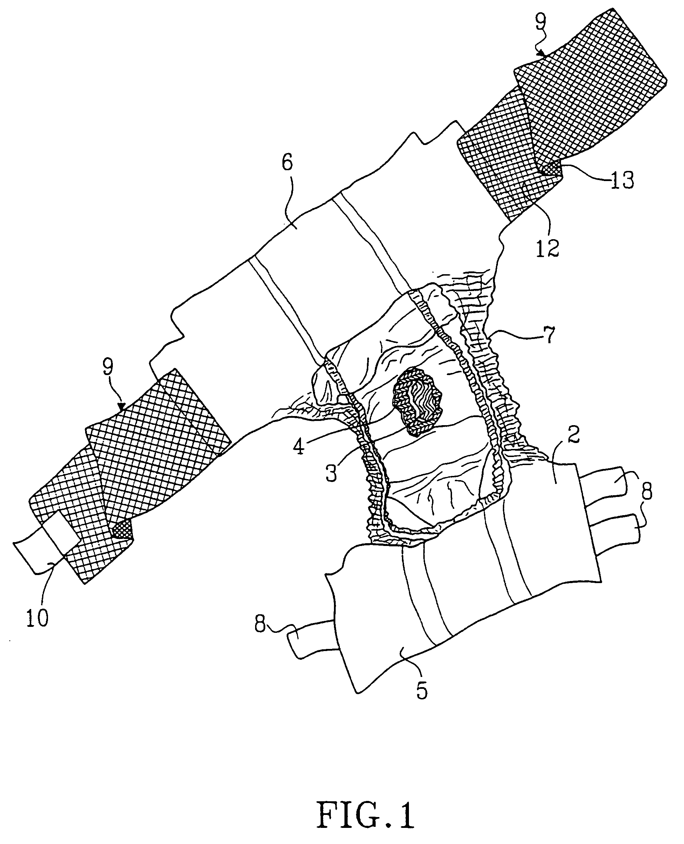

Figure 1 shows schematically a plan view of an absorbent article according to the invention.

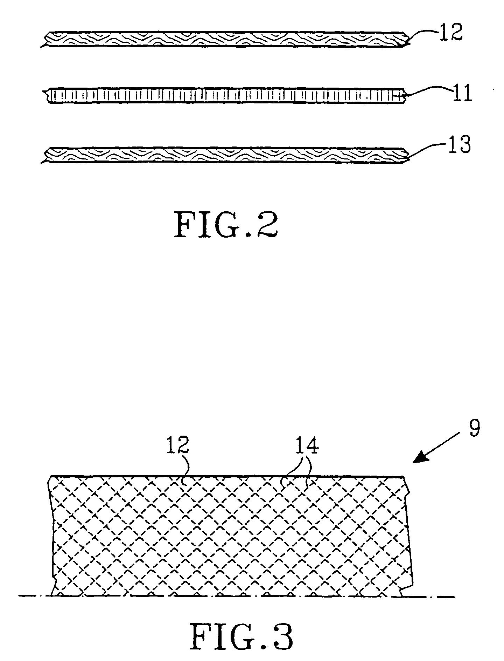

Figure 2 shows schematically an exploded view of a laminate according to the invention.
Figure 3 shows schematically the laminate seen from one side thereof.

### Description of an embodiment

The drawing shows an embodiment of a diaper or incontinence guard 1 comprising a liquid permeable topsheet 2, a liquid impermeable backsheet 3 and an absorbent body 4 enclosed therebetween. The liquid permeable topsheet 2 can consist of a nonwoven material, e g a spunbond material of continuous filaments, a meltblown material or a bonded carded fibrous web. The liquid impermeable backsheet 3 may consist of a plastic film, a nonwoven material coated with a liquid impervious material or a hydrophobic nonwoven material which resists liquid penetration.

The topsheet 2 and the backsheet material 3 has a somewhat greater extension in the plane than the absorbent body 4 and extends outside the edges thereof The layers 2 and 3 are connected to each other within the projecting portions thereof, e g by gluing or welding by heat or ultrasonic.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provided with a pair of adhesive tape tabs 8 or other type of attachment means such as hooks-and-loop type fasteners.

A pair of belt portions 9 are with one end attached, e g glued or ultrasonically welded to the rear part 5 of the diaper. The belt portions 9 are with their opposite ends intended to be fastened together, e g by a tape tab 10 which is taped to the outside of the opposite belt portion. Instead of tape there may be another type of optional attachment means, such as a hook-and-loop type fastener. The tape tabs 8 or corresponding attachment means of the front portion 5 are intended to be attached against the outside of the belt portions 9 in order to fasten together the diaper to the desired pantlike shape.

The width of the belt portions should be between 5 and 20 cm, preferably between 7 and 15 cm.

The belt portions 9 consist of an elastic laminate of an inner elastic material 11, which on both sides are covered by nonwoven materials 12, 13, one of which 12 is intended to form the outside of the belt and the other 13 is intended to form the inside of the belt, i e the side which is intended to contact the skin of the wearer. These nonwoven materials may be the same or different. The elastic carrier material 11 may, if it is soft and skin friendly enough, form the inside of the belt, at which the additional inside material 13 is eliminated.

At least the nonwoven material 12 that is intended to form the outside of the belt should be of a kind that can serve as an attachment surface for attachment means in the form of adhesive tapes as well as hook-and-loop type fasteners. These attachment means are on one hand the attachment member 10 at one belt portion 9, which is intended to be fastened to the outside of the opposite belt portion and on the other hand the attachment means 8 at the front portion of the diaper, said attachment means 8 are intended to be fastened to the outside of the belt for fastening together the diaper to the desired pant shape.

The most commonly occurring attachment means are either tape tabs or hook-and-loop type fasteners. As an attachment surface for a tape there is normally used a smooth or embossed plastic film, while a nonwoven material generally functions well as an attachment surface for the hooks in a hook-and-loop type fastener. It has now according to the invention proved that certain types of nonwoven materials also functions well as an attachment surface for a tape. It should also be possible to open and refasten the tape.

The nonwoven material should mainly consist of continuous filaments, such as spunbond material or meltblown material of for example polypropylene, polyethylene or bicomponent fibers. The basis weight of the nonwoven material which should form the attachment surface for a tape should be at least 15 and preferably at least 20 g/m², and be laminated to a carrier material, in this case the elastic material 11. Possibly the nonwoven material 12 does not need to cover the outside of the entire belt, but only the portions thereof which are intended to be used as a refastenable attachment surface for the attachment means 8 and 10.

Due to the fact that the outside of the laminate functions as an attachment surface for tapes as well as for the hooks of a hook-and-loop type fastener it is possible to use different types of attachment means 8 and 10, i e the attachment means 8 may be for example a tape while the attachment means 10 may be the hooks of a hook-and-loop type fastener. A great freedom in choice is given both regards the fit of the belt, since preferably the entire outside thereof can function as a refastenable attachment surface for the attachment means 8 and 10.

The nonwoven material that is intended to form the inside of the belt, i e directly contact the skin of the wearer, should be soft and skin friendly. A suitable nonwoven material may be the same as used as outside material 12, i e a spunbond material or a meltblown material of for example polypropylene or polyethylene. Bicomponent fibers may also be used. Another suitable nonwoven material is a carded thermobonded material of for example polypropylene, polyester- or bicomponent fibers.

As an elastic carrier material 11 there can be used an elastic film, e g of styrenebutadiene-styrene. The film can optionally be provided with holes, e g be perforated, so that it is breathable, in order to make the belt more comfortable and skin friendly. The thickness of the film is preferably in the interval 30-200 µm. Alternatively an elastic nonwoven material can be used as an elastic carrier material.

The elastic material 11 and the nonwoven material 12,13 are joined together in any suitable way, e g by gluing, heat calendering, ultrasonic welding or in another way. According to a preferred embodiment at least the nonwoven material 12 which is intended to form the outside of the belt is joined to the elastic material 11 in a pattern 14 so that a three-dimensional surface structure is formed on said side of the laminate, due to that the material has been compressed just opposite the bonding sites. Such a pattern structure 14 can for example be obtained by ultrasonic welding or heat calendering. Alternatively this may also be obtained by gluing the material layers together in a glue pattern. An improved gripping surface for the hooks of a hook-and-loop type fastener is provided by the three-dimensional structure.

The elasticity of the belt laminate is preferably obtained by the fact that also the nonwoven material 12, 13 has a certain degree of elasticity at least in one direction, the longitudinal direction of the belt. The nonwoven materials 12, 13 can also be joined to the elastic material 11 when the latter is kept in a stretched condition, at which the laminate will be elastic also in case relatively non-elastic nonwoven materials are used.

The invention is of course not limited to the above described embodiments but can be modified within the scope of the claims.

## Claims

1. Absorbent article in the form of a diaper or an incontinence guard comprising a liquid permeable topsheet (2), a liquid impermeable backsheet (3) and an absorbent body (4) enclosed therebetween, said article having a front portion (5), a rear portion (6) and a crotch portion (7) therebetween, and further is provided with a pair of belt portions (9) attached to the rear portion (6) of the article and which in use position are fastened together around the waist of the wearer by belt attachment means (10) and where said front portion (5) is provided with front portion attachment means (8) which in use position are attached to the belt portions (9), in such a way that the article will assume a pantlike shape, where the belt portions (9) form a part of the waist portions of the pant,
**characterized in**
**that** the belt portions (9) comprise a flexible laminate comprising an elastic carrier material (11), which at least on a part of the side which in use position forms the outside of the belt portions is covered with a nonwoven material (12) having a basis weight of at least 15 g/m² and which is adapted to serve as attachment surface for belt portion and front portion attachment means (8,10) both in the form of adhesive tape and hook-and-loop type fasteners, at which the attachment surface also admits refastening of said attachment means.

2. Absorbent article according to claim 1,
**characterized in**
**that** said nonwoven material (12) which in use position forms the outside of the belt mainly comprises continuous filaments, such as spunbond material or meltblown material.

3. Absorbent article according to claim 1 or 2,
**characterized in**
**that** the elastic carrier material (11) is an elastic film.

4. Absorbent article according to claim3,
**characterized in**
**that** the film (11) is perforated.

5. Absorbent article according to claims 1 or 2,
**characterized in**
**that** the elastic carrier material (11) is an elastic nonwoven material.

6. Absorbent article according to any of the preceding claims,
**characterized in**
**that** the elastic carrier material (11) on the side which in use position forms the inside of the belt is covered with a soft skin friendly nonwoven material.

7. Absorbent article according to any of the preceding claims,
**characterized in**
**that** at least the nonwoven material (12) which in use position forms the outside of the belt is joined to the elastic material (11) in a pattern (14) so that a three-dimensional surface structure is formed on said side of the laminate.

8. Absorbent article according to any of the preceding claims,
**characterized in**
**that** the nonwoven material (12) which in use position forms the outside of the belt has a basis weight of at least 20 g/m²

## Patentansprüche

1. Absorbierender Gegenstand in Form einer Windel oder eines Inkontinenzschutzes, umfassend eine flüssigkeitsdurchlässige Oberlage (2), eine flüssigkeitsundurchlässiges Rückseitenlage (3) und einen Absorptionskörper (4), der dazwischen eingeschlossen ist, wobei der Gegenstand einen Vorderabschnitt (5), einen Hinterabschnitt (6) und einen Schrittabschnitt (7) dazwischen aufweist und ferner mit einem Paar Gürtelabschnitte (9) versehen ist, die an dem Hinterabschnitt (6) des Gegenstands angebracht sind und die im Gebrauch um die Taille des Trägers durch Gürtelbefestigungseinrichtungen (10) angebracht werden, und wobei der Vorderabschnitt (5) mit einer Vorderabschnittsbefestigungseinrichtung (8) versehen ist, die im Gebrauch an den Gürtelabschnitten (9) derart angebracht wird, dass der Gegenstand eine höschenähnliche Form annehmen wird, wobei die Gürtelabschnitte (9) einen Teil der Taillenabschnitte des Höschens bilden,
**dadurch gekennzeichnet,**
**dass** die Gürtelabschnitte (9) einen flexiblen Verbund umfassen mit einem elastischen Trägermaterial (11), das auf wenigstens einem Teil der Seite, das im Gebrauch die Außenseite des Gürtelabschnitts bildet, mit einem Vliesstoff (12) bedeckt ist, der ein Basisgewicht von wenigstens 15 g/m² aufweist und geeignet ist, um als eine Befestigungsoberfläche für die Gürtelabschnittsbefestigungseinrichtung und die Vorderabschnittsbefestigungseinrichtung (8, 10) zu dienen, die beide in der Form eines Klebebands und einer Klettverschluss-Befestigungseinrichtung vorliegen, bei denen die Befestigungsoberfläche auch das Wiederbefestigen der Befestigungseinrichtung gestattet.

2. Absorbierender Gegenstand nach Anspruch 1,
**dadurch gekennzeichnet,**
**dass** der Vliesstoff (12), der im Gebrauch die Außenseite des Gürtels bildet, hauptsächlich kontinuierliche Filamente, wie beispielsweise Spunbond-Material oder Meltblown-Material umfasst.

3. Absorbierender Gegenstand nach Anspruch 1 oder 2,
**dadurch gekennzeichnet,**
**dass** das elastische Trägermaterial (11) eine elastische Folie ist.

4. Absorbierender Gegenstand nach Anspruch 3,
**dadurch gekennzeichnet,**
**dass** die Folie (11) perforiert ist.

5. Absorbierender Gegenstand nach Anspruch 1 oder 2,
**dadurch gekennzeichnet,**
**dass** das elastische Trägermaterial (11) ein elastischer Vliesstoff ist.

6. Absorbierender Gegenstand nach einem der vorstehenden Ansprüche,
**dadurch gekennzeichnet,**
**dass** das elastische Trägermaterial (11) auf der Seite, das im Gebrauch die Innenseite des Gürtels bildet, mit einem weichen und hautfreundlichen Vliesstoff bedeckt ist.

7. Absorbierender Gegenstand nach einem der vorstehenden Ansprüche,
**dadurch gekennzeichnet,**
**dass** wenigstens der Vliesstoff (12), der im Gebrauch die Außenseite des Gürtels bildet, in einem Muster (14) mit dem elastischen Material (11) verbunden ist, so dass eine dreidimensionale Oberflächenstruktur auf der besagten Seite des Verbunds ausgebildet wird.

8. Absorbierender Gegenstand nach einem der vorstehenden Ansprüche,
**dadurch gekennzeichnet,**
**dass** der Vliesstoff (12), der im Gebrauch die Außenseite des Gürtels bildet, ein Flächengewicht von wenigstens 20 g/m2 aufweist.

## Revendications

1. Article absorbant sous la forme d'une couche-culotte ou d'une protection contre l'incontinence comprenant une feuille avant (2) perméable aux liquides, une feuille arrière (3) imperméable aux liquides et un corps absorbant (4) enfermé entre elles, ledit article comportant une partie avant (5), une partie arrière (6) et une partie entrejambe (7) entre elles, et muni en outre d'une paire de parties de ceinture (9) attachées à la partie arrière (6) de l'article et qui, en position d'utilisation, sont fixées l'une à l'autre autour de la taille de l'utilisateur à l'aide d'un moyen de fermeture (10) de ceinture et où ladite partie avant (5) est munie de moyens de fermeture (8) de partie avant qui, en position d'utilisation, sont attachés aux parties de ceinture (9), de manière telle que l'article prend une forme de culotte, où les parties de ceinture (9) forment une partie des parties de taille de la culotte,
**caractérisé en ce que** les parties de ceinture (9) comprennent un stratifié souple comprenant un matériau de support élastique (11), qui, au moins sur une partie du côté qui en position d'utilisation forme l'extérieur des parties de ceinture, est recouvert d'un matériau non tissé (12) ayant une masse surfacique d'au moins 15 g/m² et qui est adapté pour servir de surface de fixation aux moyens de fermeture de partie de ceinture et de partie avant (8, 10), à la fois sous la forme de ruban adhésif et de dispositifs de fermeture du type à crochets et à boucles, au niveau desquels la surface de fixation permet aussi une refixation desdits moyens de fermeture.

2. Article absorbant selon la revendication 1, **caractérisé en ce que** ledit matériau non tissé (12) qui, en position d'utilisation, forme l'extérieur de la ceinture, comprend principalement des filaments continus, comme un matériau filé-lié ou un matériau fondu-soufflé.

3. Article absorbant selon la revendication 1 ou 2, **caractérisé en ce que** le matériau de support élastique (11) est un film élastique.

4. Article absorbant selon la revendication 3, **caractérisé en ce que** le film (11) est perforé.

5. Article absorbant selon la revendication 1 ou 2, **caractérisé en ce que** le matériau de support élastique (11) est un matériau non tissé élastique.

6. Article absorbant selon l'une quelconque des revendications précédentes, **caractérisé en ce que** le matériau de support élastique (11), sur le côté qui en position d'utilisation forme l'intérieur de la ceinture, est recouvert d'un matériau non tissé doux et non agressif pour la peau.

7. Article absorbant selon l'une quelconque des revendications précédentes, **caractérisé en ce qu'**au moins le matériau non tissé (12) qui en position d'utilisation forme l'extérieur de la ceinture est relié au matériau élastique (11) suivant un motif (14) de telle manière qu'une structure de surface tridimensionnelle est formée sur ledit côté du stratifié.

8. Article absorbant selon l'une quelconque des revendications précédentes, **caractérisé en ce que** le matériau non tissé (12) qui en position d'utilisation forme l'extérieur de la ceinture a une masse surfacique d'au moins 20 g/m².
